(19) **Europäisches Patentamt / European Patent Office / Office européen des brevets**

(11) **EP 3 597 660 A1**

(12) **EUROPEAN PATENT APPLICATION**
published in accordance with Art. 153(4) EPC

(43) Date of publication:
**22.01.2020 Bulletin 2020/04**

(21) Application number: **18767734.9**

(22) Date of filing: **16.03.2018**

(51) Int Cl.:
*C07K 1/14* (2006.01)  *A61K 35/19* (2015.01)
*A61M 1/02* (2006.01)  *A61P 7/00* (2006.01)
*C12M 1/00* (2006.01)  *C07K 14/78* (2006.01)
*C12N 15/09* (2006.01)

(86) International application number:
**PCT/JP2018/010508**

(87) International publication number:
**WO 2018/169061 (20.09.2018 Gazette 2018/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.03.2017 JP 2017050825**

(71) Applicant: **FUJIFILM Corporation
Tokyo 106-8620 (JP)**

(72) Inventors:
• **TAKEI, Toshiki**
  **Ashigarakami-gun**
  **Kanagawa 258-8577 (JP)**
• **YAMADA, Tadanori**
  **Ashigarakami-gun**
  **Kanagawa 258-8577 (JP)**

(74) Representative: **Hoffmann Eitle
Patent- und Rechtsanwälte PartmbB
Arabellastraße 30
81925 München (DE)**

(54) **METHOD FOR SEPARATING MEGAKARYOCYTES AND PLATELETS AND INSTRUMENT FOR SEPARATING MEGAKARYOCYTES AND PLATELETS**

(57)     An object of the present invention is to provide a method for efficiently separating megakaryocytes and platelets produced from the megakaryocytes, and an instrument for efficiently separating megakaryocytes and platelets produced from the megakaryocytes. According to the present invention, a method for separating megakaryocytes and platelets, including a contact step of bringing a culture solution that contains at least megakaryocytes into contact with a substrate coated with a biocompatible polymer that adheres to the megakaryocytes via at least one of a VLA-4 integrin or a VLA-5 integrin; a culture step of culturing the megakaryocytes to produce platelets before and/or after the contact step; and a recovery step of recovering the culture solution after the contact step and the culture step is provided.

EP 3 597 660 A1

**Description**

**BACKGROUND OF THE INVENTION**

1. Field of the Invention

The present invention relates to a method for separating megakaryocytes and platelets produced from the megakaryocytes. The present invention further relates to an instrument for separating megakaryocytes and platelets.

2. Description of the Related Art

Platelets are used in blood transfusion therapy as a platelet formulation. A platelet formulation is generally prepared from blood obtained by blood donation, but a supply amount of platelet formulations is likely to be influenced by external factors such as a reduction in blood donors. Accordingly, attention has been focused on the development of platelet sources that can replace blood donations. In recent years, a technology has been reported for mass production of platelets in vitro by culturing megakaryocytes with use of pluripotent stem cells, hematopoietic precursor cells, and mesenchymal lineage cells as sources (Patent Document 1). Platelets are produced by breaking up the cytoplasm of megakaryocytes, and therefore a culture solution after platelet production contains a large number of megakaryocytes. There is a possibility that a platelet formulation has immunogenicity due to inclusion of megakaryocytes. Therefore, it is necessary to develop a technology for separating megakaryocytes and platelets produced from the megakaryocytes.

As a method for separating cells, a centrifugation method for separating cells based on a difference in specific gravity of cells is generally used. For example, Patent Document 2 discloses a platelet collection device including a centrifuge that has, in the inside thereof, a blood storage space communicating with an inlet port and an outlet port; a first line that is connected to the inlet port of the centrifuge and a blood collection means; a second line that is connected to the outlet port of the centrifuge; a plasma collection bag that has a first tube connected in the middle of the first line and a second tube connected to the second line; a platelet collection circuit that has a platelet collection bag connected to the second line; and a blood feeding pump that is provided in the first line, in which the blood collected by the centrifuge is separated into a plurality of blood components, and at least platelets of the separated blood components are collected in the platelet bag.

In addition, a method in which, in a case of removing leukocytes that has a probability of exhibiting immunogenicity from a platelet formulation, platelets are separated by allowing a liquid containing platelets and leukocytes to permeate through a porous membrane having a pore diameter that allows platelets to permeate through and does not allow leukocytes to permeate through by utilizing a difference in size between leukocytes and platelets is known (Patent Document 3).

Furthermore, in a case of producing a platelet formulation from blood, platelets are separated by removing leucocytes contained in a platelet formulation by using an adsorptive separation material that selectively absorbs leucocytes (Patent Document 4).

Prior Art Documents

Patent Documents

Patent Document 1: WO09/122747A

Patent Document 2: JP2003-093499A

Patent Document 3: WO93/024157

Patent Document 4: JP2000-245833A

**SUMMARY OF THE INVENTION**

Unlike a case of separation of a platelet component from blood components, it is necessary to remove megakaryocytes having a density close to that of platelets in order to separate and recover platelets produced in vitro. Accordingly, in a case of separating platelets by using a centrifugal separation method as disclosed in Patent Document 2, a large centrifugal acceleration is required, and there is a problem of activation of platelets due to centrifugal force.

In addition, in a case of removing megakaryocytes, megakaryocytes produce platelets, and a size of a megakaryocyte itself is reduced. Therefore, megakaryocytes of which a size difference from platelets is not large may be present among megakaryocytes after producing platelets. For this reason, in methods utilizing a difference in cell size as in the method disclosed in Patent Document 3, there is a problem of a decrease in removal percentage of megakaryocytes, and also a problem of damage on platelets in a case where platelets permeate through a membrane.

Furthermore, the method disclosed in Patent Document 4 is a method in which a target to be separated from platelets is a leucocyte, and thus is not a method for separating platelets from megakaryocytes.

An object of the present invention is to provide a method for efficiently separating megakaryocytes and platelets produced from the megakaryocytes, and an instrument for efficiently separating megakaryocytes and platelets produced from the megakaryocytes.

As a result of intensive studies to solve the above-described problems, the inventors of the present invention have found that megakaryocytes and platelets can be separated by using a material that selectively absorbs megakaryocytes, and therefore have completed the present invention.

In other words, according to the present invention, the following invention is provided.

[1] A method for separating megakaryocytes and platelets, the method comprising:

a contact step of bringing a culture solution that contains at least megakaryocytes into contact with a substrate coated with a biocompatible polymer that adheres to the megakaryocytes via at least one of a VLA-4 integrin or a VLA-5 integrin;
a culture step of culturing the megakaryocytes to produce platelets before and/or after the contact step; and
a recovery step of recovering the culture solution after the contact step and the culture step.

[2] The method according to [1], in which megakaryocytes are cultured to produce platelets, a culture solution that contains the obtained megakaryocytes and the platelets is brought into contact with the substrate coated with the biocompatible polymer that adheres to the megakaryocytes via at least one of a VLA-4 integrin or a VLA-5 integrin, and the culture solution brought into contact with the substrate is recovered.

[3] The method according to [1], in which the culture solution that contains the megakaryocytes is brought into contact with the substrate coated with the biocompatible polymer that adheres to the megakaryocytes via at least one of a VLA-4 integrin or a VLA-5 integrin, the megakaryocytes brought into contact with the substrate are cultured to produce platelets, and the culture solution is recovered.

[4] The method according to any one of [1] to [3], in which the biocompatible polymer that adheres to the megakaryocytes via at least one of a VLA-4 integrin or a VLA-5 integrin is a fibronectin; a peptide having GRGDS that is an RGD sequence or EILDV that is a CS-1 sequence of the fibronectin; Vascular Cell Adhesion Molecule-1 represented as VCAM-1; or a peptide having a QIDSPL sequence of VCAM-1.

[5] The method according to any one of [1] to [4], in which the contact step, the culture step, and the recovery step are repeated twice or more.

[6] An instrument for separating megakaryocytes and platelets, the instrument comprising a substrate coated with a biocompatible polymer that adheres to megakaryocytes via at least one of a VLA-4 integrin or a VLA-5 integrin.

According to the method and the instrument of the present invention, megakaryocytes and platelets produced from the megakaryocytes can be efficiently separated.

## DESCRIPTION OF THE PREFERRED EMBODIMENTS

Hereinafter, the embodiment of the present invention will be described in detail.

A method for separating megakaryocytes and platelets according to the embodiment of the present invention, includes
a contact step of bringing a culture solution that contains at least megakaryocytes into contact with a substrate coated with a biocompatible polymer that adheres to the megakaryocytes via at least one of a VLA-4 integrin or a VLA-5 integrin;
a culture step of culturing the megakaryocytes to produce platelets before and/or after the contact step; and
a recovery step of recovering the culture solution after the contact step and the culture step.

In manufacturing a platelet formulation used for blood transfusion therapy and the like, the method of the embodiment of the present invention can be used as a method for separating platelets in a case where a large amount of platelets are produced in vitro.

<Regarding megakaryocytes and platelets>

Megakaryocytes are cells derived from hematopoietic stem cells, and are cells having a polymorphonuclear nucleus formed via megakaryocyte precursor cells, megakaryoblasts, and promegakaryocytes. Megakaryocytes are present in the bone marrow and are the largest hematopoietic lineage cells having a diameter of 35 to 160 μm in the bone marrow. Megakaryocytes produce platelets. Thousands of platelets are produced from per megakaryocyte. After the cytoplasm of megakaryocytes is degraded to become platelets, the megakaryocytes lose their cytoplasm and become naked nuclei, and the naked nuclei are degraded by macrophages. Megakaryocytes referred to in the embodiment of the present invention may include, megakaryocyte precursors, megakaryoblasts, promegakaryocytes, and the like, in addition to mature megakaryocytes.

Platelets are a type of cellular component contained in blood. Platelets play a central role in thrombus formation, and have the action of hemostasis by platelet aggregation in a case where the vascular wall is damaged.

Megakaryocytes and platelets may be megakaryocytes and platelets collected from adult tissues, may be megakaryocytes and platelets differentiated from cells having a differentiation capacity, such as pluripotent stem cells, hematopoietic precursor cells, and mesenchymal lineage cells, may be megakaryocytes and platelets produced by using a direct reprogramming technique for cells that do not have a capacity to be differentiated into megakaryocytes by general methods, or may be a combination of the above megakaryocytes and platelets.

An organism species from which megakaryocytes and platelets are derived is not particularly limited, but is preferably mammals (for example, humans, mice, rats, hamsters, guinea pigs, sheep, pigs, monkeys, and the like), and is more preferably humans.

Examples of pluripotent stem cells include embryonic stem cells (ES cells), nuclear transfer embryonic stem cells (ntES cells), induced pluripotent stem cell (iPS cells), and the like, but are not limited thereto.

Examples of hematopoietic precursor cells include cells derived from bone marrow, cells derived from umbilical cord blood, cells derived from (G-CSF)-mobilized peripheral blood, middle lobe lung cells derived from ES cells, cells derived from peripheral blood, and the like, but are not limited thereto. Examples of these hematopoietic precursor cells include cells positive to CD34 (for example, CD34+ cells, CD133+ cells, SP cells, CD34+ CD38-cells, c-kit+ cells, or CD3-, CD4-, CD8-, and CD34+ cells) (WO2004/110139A).

Examples of mesenchymal lineage cells include mesenchymal lineage stem cells, adipocyte precursor cells, and the like, but are not limited thereto.

Examples of cells that do not have a capacity to be differentiated into megakaryocytes by general methods include fibroblasts and the like, but are not limited thereto.

It is sufficient that a method for producing megakaryocytes and platelets by differentiating cells having a differentiation capacity, such as pluripotent stem cells, hematopoietic precursor cells, and mesenchymal lineage cells is performed according to methods generally known to those skilled in the art, and the method is not particularly limited. By culturing cells having a differentiation capacity under appropriate culture conditions by using a differentiation-inducing medium suitable for differentiating the cells into megakaryocytes, it is possible to differentiate the cells having a differentiation capacity into megakaryocytes, and platelets are produced from the megakaryocytes. As a method for producing megakaryocytes and platelets by using a direct reprogramming technique for cells that do not have a capacity to be differentiated into megakaryocytes by the general methods, an induction gene may be introduced into cells so that the induction gene into megakaryocytes is expressed; or a specific nucleic acid, protein, low molecular weight compound, or the like may be added to a culture solution to differentiate, into megakaryocytes, the cells that do not a capacity to be differentiated into megakaryocytes by the general methods. Therefore, it is possible to differentiate the cells into megakaryocytes.

<Contact step>

The contact step is a step of bringing a culture solution that contains at least megakaryocytes into contact with a substrate coated with a biocompatible polymer that adheres to the megakaryocytes via at least one of a VLA-4 integrin or a VLA-5 integrin.

The type of the "biocompatible polymer that adheres to megakaryocytes via at least one of a VLA-4 integrin or a VLA-5 integrin" is not particularly limited as long as the object of the present invention can be achieved, and specific examples thereof are as follows.

Fibronectin;

A peptide having a RGD sequence (GRGDS: SEQ ID NO: 1) or a CS-1 sequence (EILDV: SEQ ID NO: 2) (VLA-4 integrin- or VLA-5 integrin-adhesion domain of fibronectin) of a fibronectin; Vascular Cell Adhesion Molecule-1 (VCAM-1);

A peptide having a QIDSPL sequence (SEQ ID NO: 3) (VLA-4 integrin- or VLA-5 integrin-adhesion domain of VCAM-1) of VCAM-1;

Mucosal adressin cell adhesion molecule-1 (MadCAM-1);

Thrombospondin (TSP);

Osteopontin (OPN);

A Disintegrin and Mettalloprotease (ADAM);

Intercellular adhesion molecule-1 (ICAM-4);

Cartilage oligomeric matrix (COMP); and

L1 (CD171)

Among the above examples, a fibronectin; a peptide having a RGD sequence (GRGDS) or a CS-1 sequence (EILDV) of a fibronectin; VCAM-1; or a peptide having a QIDSPL sequence of VCAM-1 is preferable.

Among the above examples, a fibronectin or Vascular Cell Adhesion Molecule-1 (VCAM-1) is more preferable.

In a case of using a peptide, a length of the peptide is not particularly limited, but generally, it is possible to use a peptide consisting of 5 amino acids to 200 amino acids, and preferably, it is possible to use a peptide consisting of 5 amino acids to 50 amino acids.

The above-mentioned biocompatible polymer may be any of a natural protein, a recombinant protein or a recombinant peptide, or a chemically synthesized protein or a chemically synthesized peptide.

As natural proteins, it is possible to use proteins derived from organs, tissues, or cells of various organisms.

Recombinant proteins or recombinant peptides can be manufactured by recombinant technology known to those skilled in the art, and can be manufactured according to, for example, methods disclosed in EP1014176A2, US6992172B, WO2004/085473A, WO2008/103041A, and the like. Specifically, a gene encoding a desired amino acid sequence is obtained and incorporated into an expression vector to produce a recombinant expression vector, which is introduced into a suitable host to produce a transformant. By culturing the obtained transformant in an appropriate medium, a recombinant protein or a recombinant peptide is produced. By recovering the produced recombinant protein or recombinant peptide from the culture, it is possible to obtain a recombinant protein or a recombinant peptide.

A chemically synthesized protein or a chemically synthesized peptide means an artificially synthesized protein or peptide. The synthesis of protein or peptide may be solid phase synthesis or liquid phase synthesis, but is preferably solid phase synthesis. Solid phase synthesis of peptides is known to those skilled in the art. Examples thereof include Fmoc group synthesis using Fmoc group (Fluorenyl-Methoxy-Carbonyl group) as protection of amino group; Boc group synthesis method using Boc group (tert-Butyl Oxy Carbonyl group) as protection of amino group; and the like.

Examples of substrates include plates, dishes, petri dishes, culture flasks, particles, non-woven fabrics, membranes, and the like. As the plate, a multiwell plate (for example, a 6-well plate, a 24-well plate, and the like) may be used.

A method for coating a substrate with the above-mentioned biocompatible polymer is not particularly limited, and the substrate can be coated with the biocompatible polymer by general methods. For example, a solution of a biocompatible polymer is prepared as a coating solution at an appropriate concentration (for example, 0.001 to 1000 $\mu$g/ml, preferably 1 to 100 $\mu$g/ml), this coating solution is added to a substrate, the substrate is incubated at an appropriate temperature (for example, room temperature to 50°C, for example 37°C) for a certain time (for example, 10 minutes to 24 hours, preferably 30 minutes to 6 hours), and therefore a biocompatible polymer-coated substrate can be prepared. After the above-described incubation, a biocompatible polymer-coated substrate may be washed with an appropriate buffer solution.

A method for bringing a culture solution containing at least megakaryocytes into contact with a biocompatible polymer-coated substrate is not particularly limited, and can be carried out by general methods. For example, a culture solution containing at least megakaryocytes can be added to a biocompatible polymer-coated substrate to bring, into contact with the culture solution, the substrate at a suitable temperature (for example, room temperature to 50°C, for example 37°C) for a certain period of time (for example, 24 hours or less, preferably 6 hours or less in a case of culturing megakaryocytes to produce platelets before the contact step, and for example, 1 day to 7 days, preferably 1 day to 4 day in a case of culturing megakaryocytes to produce platelets after the contact step).

<Culture step of culturing megakaryocytes in culture solution to produce platelets>

In the present invention, a culture step of culturing the megakaryocytes to produce platelets is performed before and/or after the contact step. In other words, in the present invention, separation of megakaryocyte and platelets may be applied with respect to a mixed solution of megakaryocytes and platelets after megakaryocytes are cultured to produce platelets in advance. Alternatively, megakaryocytes are brought into contact with a substrate coated with the biocompatible polymer that adheres to the megakaryocytes via at least one of a VLA-4 integrin or a VLA-5 integrin, and separation of megakaryocyte and platelets may be applied while allowing the megakaryocytes to produce platelets in a state in which the megakaryocytes and the substrate are adhered.

Accordingly, the present invention may be any of the following aspects.

(Aspect 1) Megakaryocytes are cultured to produce platelets, a culture solution that contains the obtained megakaryocytes and the platelets is brought into contact with a substrate coated with a biocompatible polymer that adheres to the megakaryocytes via at least one of a VLA-4 integrin or a VLA-5 integrin, and the culture solution brought into contact with the substrate is recovered.

(Aspect 2) A culture solution that contains megakaryocytes is brought into contact with a substrate coated with a biocompatible polymer that adheres to the megakaryocytes via at least one of a VLA-4 integrin or a VLA-5 integrin, the megakaryocytes brought into contact with the substrate are cultured to produce platelets, and the culture solution is recovered.

(Aspect 3) Megakaryocytes are cultured to produce platelets, a culture solution that contains the obtained megakaryocytes and the platelets is brought into contact with a substrate coated with a biocompatible polymer that adheres to the megakaryocytes via at least one of a VLA-4 integrin or a VLA-5 integrin, megakaryocytes brought into contact with the substrate are further cultured to produce platelets, and then the culture solution is recovered.

Among the above aspects, the aspect 1 or the aspect 2 is preferable, and the aspect 1 is more preferable.

The culture step of culturing megakaryocytes in a culture solution to produce platelets can be performed according to general methods for culturing cells. A culture period is not particularly limited, but is generally 3 hours to 30 days, is preferably 24 hours to 20 days, and is more preferably about 3 days to 14 days.

<Recovery step of recovering culture solution after contact step and culture step>

Platelets have lower adhesiveness with respect to a substrate coated with a biocompatible polymer that adheres to the megakaryocytes via at least one of a VLA-4 integrin or a VLA-5 integrin than that of megakaryocytes, and therefore platelets can be efficiently recovered by the step of recovering a culture solution brought into contact with a substrate.

In order to check an abundance (the number of cells) of megakaryocytes and platelets in a recovered solution, the recovered solution can be reacted with a labeled anti-CD41a antibody and then reacted with Hoechst 33342 which is a nuclear stain agent, and flow cytometry can be performed. Megakaryocyte fractions and platelet fractions are determined, and CD41-positive cells and nuclear stain negative cells in the platelet fractions are perceived as platelets, and CD41-positive cells and nuclear stain positive cells in the megakaryocyte fractions are perceived as megakaryocytes. Therefore, the number of platelets and the number of megakaryocytes in the recovered solution can be calculated.

In the present invention, the contact step, the culture step, and the recovery step can be repeated twice or more. A recovery percentage of platelets can be further improved by repeating the contact step, the culture step, and the recovery step twice or more. The number of repetitions is not particularly limited, but may be, for example, about 2 to 10 times.

<Instrument for separating megakaryocytes and platelets>

According to the present invention, an instrument for separating megakaryocytes and platelets, including a substrate coated with a biocompatible polymer that adheres to megakaryocytes via at least one of a VLA-4 integrin or a VLA-5 integrin, is provided. As a substrate coated with a biocompatible polymer that adheres to megakaryocytes via at least one of a VLA-4 integrin or a VLA-5 integrin, it is possible to use the substrate described above in the present specification. A method for separating megakaryocytes and platelets by using the above-mentioned instrument of the embodiment of the present invention is also as described above in the present specification.

The present invention will be described more specifically by the following examples, but the present invention is not limited by the examples.

The following abbreviations have the following meanings.

FBS: Fetal bovine serum

DPBS: Dulbecco's Phosphate-Buffered Saline

PE: Phycoerythrin

7-AAD: 7-amino-7- actinomycin D

Examples

<Example 1>

(1) Coating on substrate

A fibronectin solution (fibronectin, derived from human plasma, manufactured by Wako Pure Chemical Industries, Ltd.) was diluted to 50 $\mu$g/ml with DPBS (manufactured by Thermo Fisher Scientific) to be used as a coating solution. 1 ml of the coating solution was added to plasma-treated 6-well polystyrene plate (Tissue Culture-Treated, manufactured by Corning Incorporated). The plate was incubated at 37°C for 1 hour, and then washed once with DPBS, and therefore a fibronectin-coated substrate was obtained.

(2) Preparation of cell fluid

StemSpan SFEM II (manufactured by STEMCELL Technologies Inc.) into which StemSpan megakaryocyte expansion supplement (manufactured by STEMCELL Technologies Inc.) and Penicillin-Streptomycin Solution ($\times$ 100) (manufactured by Wako Pure Chemical Industries) were added was used as a differentiation-inducing medium for preparing human megakaryocytes and platelets. By using the above-described differentiation-inducing medium, BM-CD34$^+$ Stem/Progenitor Cells (Allcell) were cultured for 14 days on a low adherent 6-well polystyrene plate (Ultra-Low Attachment Surface, manufactured by Corning Incorporated). The floating cells in culture were recovered in a 15 mL tube (manufactured by Corning Incorporated) and centrifuged at 1700 $\times$ g for 5 minutes at room temperature. The supernatant was discarded and the cells were suspended in DPBS (100 $\mu$l) containing 1% FBS (manufactured by Thermo Fisher Scientific). 10 $\mu$l of PerCP-Cy5.5 (registered trademark)-labeled anti-CD41a antibody (manufactured by Nippon Becton, Dickinson and Company) and 10 $\mu$l of PE-labeled anti-CD42b antibody (manufactured by Nippon Becton, Dickinson and Company) were added, and reaction was performed for 30 minutes with light shielding. DPBS (1 mL) containing 1% FBS was added, the mixture was centrifuged at 1700 $\times$ g for 5 minutes at room temperature, and the supernatant was discarded. The cells were suspended in DPBS (100 $\mu$l) containing 1% FBS to which 7-AAD was added, reacted for 15 minutes with light shielding, DPBS (300 $\mu$l) containing 1% FBS was added thereto, and measurement was performed by flow cytometry (manufactured by Becton, Dickinson and Company (BD), FACS Aria). Megakaryocyte fractions and platelet fractions were determined from a forward-scattered light (FSC) by side-scattered light (SSC) gate with use of MEG-01 (purchased from American Type Culture Collection (ATCC)), which is a megakaryocyte cell line, as a control. The presence of CD41a-positive cells and CD42b-positive cells was checked by comparison of each antibody with a sample stained with isotype control antibodies, and therefore megakaryocyte differentiation from BM-CD34$^+$ Stem/Progenitor Cells and platelet production from the megakaryocytes were confirmed.

(3) Contact test of platelets

The culture solution of human megakaryocytes and platelets which was obtained by the method of (2) described above was allowed to pass through a 40 $\mu$m cell strainer (manufactured by Falcon). Thereafter, 2 ml of the culture solution was added to the fibronectin-coated substrate prepared in (1) described above per well, and contacted at 37°C for 1 hour. After lapse of one hour, the entire supernatant of the culture solution was recovered in a 15 mL conical tube for centrifugation (manufactured by Falcon) which contains a citrate dextrose solution (ACD) (manufactured by Sigma-Aldrich). In addition, by performing washing twice with DPBS and recovering the wash fluid in a conical tube, floating cells not absorbed to the fibronectin-coated substrate were recovered.

(4) Counting the number of recovered cells

The recovered solution was recovered in a 15 mL tube and centrifuged at 1700 x g at room temperature for 10 minutes. The supernatant was discarded and the cells were suspended in DPBS (100 $\mu$l) containing 1% FBS. 10 $\mu$l of PerCP-Cy5.5 (registered trademark)-labeled anti-CD41a antibody was added and reacted for 30 minutes with light shielding. DPBS (1 mL) containing 1% FBS was added, and the mixture was centrifuged at 1700 $\times$ g for 10 minutes at room temperature, and the supernatant was discarded. The cells were suspended in DPBS (100 $\mu$L) containing 1% FBS into which Hoechst 33342 (manufactured by Dojin Chemical Research Institute), which is a nuclear stain agent, was added, and reacted for 15 minutes in a light-shielding environment. DPBS (300 $\mu$L) containing 1% FBS was added thereto, and measurement was performed by flow cytometry (FACS Aria) by using BD Trucount tubes (manufactured by Nippon Becton, Dickinson and Company). Megakaryocyte fractions and platelet fractions were determined from a FSC by SSC gate with use of MEG-01 which is a megakaryocyte cell line as a control. CD41-positive cells and nuclear stain negative cells in the platelet fractions are perceived as platelets, and CD41-positive cells and nuclear stain positive cells in the megakaryocyte fractions are perceived as megakaryocytes. Therefore, the number of platelets and the number of megakaryocytes in the recovered solution was calculated. A recovery percentage of platelets and a recovery percentage of megakaryocytes obtained from the following equation are shown in Table 1.

$$\text{Recovery percentage of platelets (\%)} = (\text{the number of platelets in recovered solution}$$
$$\text{obtained after contact with substrate/the number of platelets in original solution}) \times 100$$

Recovery percentage of megakaryocytes (%) = (the number of megakaryocytes in recovered solution obtained after contact with substrate/the number of megakaryocytes in original solution) × 100

A separation performance of platelets obtained by contact with substrate was classified based on the following index.

Index value = recovery percentage of platelets (%)/recovery percentage of megakaryocytes (%)

Separation performance A: index value > 10.0
Separation performance B: 10.0 ≥ index value > 5.0
Separation performance C: 5.0 ≥ index value > 2.5
Separation performance D: 2.5 ≥ index value

<Examples 2 and 3>

The same operation as in Example 1 was carried out except that a fibronectin (rfibronectin, human, recombinant, manufactured by R & D) was diluted to 50 μg/ml with DPBS to be used as a coating solution (Example 2), and VCAM-1 (human, recombinant, manufactured by Wako Pure Chemical Industries, Ltd.) was diluted to 5 μg/ml with DPBS to be used as a coating solution (Example 3). The results are shown in Table 1.

<Comparative Examples 1 to 6>

As comparative examples, a fibrinogen (derived from human plasma, manufactured by Wako Pure Chemical Industries, Ltd., Comparative Example 1), collagen I (derived from a rat tail, manufactured by Corning Incorporated, Comparative Example 2), collagen IV (derived from a human placenta, manufactured by Sigma-Aldrich, Comparative Example 3), Laminin (derived from a human umbilical cord, manufactured by Sigma-Aldrich, Comparative Example 4), and vwf (von Willebranf Factor, derived from human plasma, manufactured by Haematologic Technologies, Comparative Example 5) were used as coating solutions in experiments. In addition, as Comparative Example 6, DPBS was used instead of the coating solution. The other operations were performed in the same manner as in Example 1. The results are shown in Table 1.

[Table 1]

|  | Coated substrate | Recovery percentage of platelets (%) | Recovery percentage of megakaryocytes (%) | Recovery percentage of platelets (%)/recovery percentage of megakaryocytes (%) | Separation performance |
|---|---|---|---|---|---|
| Example 1 | fibronectin | 80.2% | 11.4% | 7.0 | B |
| Example 2 | rfibronectin | 67.3% | 6.2% | 10.8 | A |
| Example 3 | VCAM-1 | 63.0% | 5.9% | 10.6 | A |
| Comparative Example 1 | fibrinogen | 73.3% | 53.7% | 1.4 | D |
| Comparative Example 2 | collagen I | 75.9% | 47.0% | 1.6 | D |
| Comparative Example 3 | collagen IV | 81.8% | 51.3% | 1.6 | D |
| Comparative Example 4 | Laminin | 79.9% | 44.0% | 1.8 | D |

(continued)

|  | Coated substrate | Recovery percentage of platelets (%) | Recovery percentage of megakaryocytes (%) | Recovery percentage of platelets (%)/recovery percentage of megakaryocytes (%) | Separation performance |
|---|---|---|---|---|---|
| Comparative Example 5 | vwf | 63.3% | 19.7% | 3.2 | C |
| Comparative Example 6 | Not coated | 69.0% | 67.1% | 1.0 | D |

As shown in the results of Examples 1 to 3, in a case of using the separation method of the embodiment of the present invention, a recovery percentage of platelets to a recovery percentage of megakaryocytes is high, and therefore platelets can be efficiently separated and recovered.

<Examples 4 and 5>

A step of bringing a culture solution of suspended cells after brought into contact with the fibronectin-coated substrate for 1 hour in Example 1 into contact with another fibronectin-coated substrate for 1 hour was repeated multiple times. The other operations were performed in the same manner as in Example 1. The results are shown in Table 2.

[Table 2]

|  | Coated substrate | Number of times of contact | Recovery percentage of platelets (%) | Recovery percentage of megakaryocytes (%) | Recovery percentage of platelets (%)/recovery percentage of megakaryocytes (%) | Separation performance |
|---|---|---|---|---|---|---|
| Example 4 | fibronectin | 2 | 87.1% | 8.6% | 10.1 | A |
| Example 5 | fibronectin | 3 | 88.5% | 8.3% | 10.6 | A |

As shown in the results of Examples 4 and 5, it was possible to improve a separation performance of platelets by repeating the separation method of the embodiment of the present invention multiple times.

Sequence Listing

International Application 17F02661 Method for separating megakaryocytes and platelets based on the International Patent Cooperation Treaty JP18010508 20180316----00020006351800558056 normal 20180316151008201802281059523640_P1AP101__17_2.app

```
SEQUENCE LISTING
<110>  FUJIFILM Corporation
<120>  A method for separation of platelet from megakaryocyte, and a device
for separation of platelet from megakaryocyte
<130>\201@17F02661
<160>  3
<170>  PatentIn version 3.5
<210>  1
<211>  5
<212>  PRT
<213>  Artificial Sequence
<220>
<223>  Description of Artificial Sequence: adhesive sequence
<400>  1
Gly Arg Gly Asp Ser
1               5
<210>  2
<211>  5
<212>  PRT
<213>  Artificial Sequence
<220>
<223>  Description of Artificial Sequence: adhesive sequence
<400>  2
Glu Ile Leu Asp Val
1               5
<210>  3
<211>  6
<212>  PRT
<213>  Artificial Sequence
<220>
<223>  Description of Artificial Sequence: adhesive sequence
400>   3
Gln Ile Asp Ser Pro Leu
1               5
```

**Claims**

1. A method for separating megakaryocytes and platelets, the method comprising:

   a contact step of bringing a culture solution that contains at least megakaryocytes into contact with a substrate coated with a biocompatible polymer that adheres to the megakaryocytes via at least one of a VLA-4 integrin or a VLA-5 integrin;
   a culture step of culturing the megakaryocytes to produce platelets before and/or after the contact step; and
   a recovery step of recovering the culture solution after the contact step and the culture step.

2. The method according to claim 1,

   wherein megakaryocytes are cultured to produce platelets,
   a culture solution that contains the obtained megakaryocytes and the platelets is brought into contact with the substrate coated with the biocompatible polymer that adheres to the megakaryocytes via at least one of a VLA-4 integrin or a VLA-5 integrin, and
   the culture solution brought into contact with the substrate is recovered.

3. The method according to claim 1,

   wherein the culture solution that contains the megakaryocytes is brought into contact with the substrate coated with the biocompatible polymer that adheres to the megakaryocytes via at least one of a VLA-4 integrin or a VLA-5 integrin,
   the megakaryocytes brought into contact with the substrate are cultured to produce platelets, and

the culture solution is recovered.

4. The method according to any one of claims 1 to 3, wherein the biocompatible polymer that adheres to the megakaryocytes via at least one of a VLA-4 integrin or a VLA-5 integrin is a fibronectin; a peptide having GRGDS that is an RGD sequence or EILDV that is a CS-1 sequence of the fibronectin; Vascular Cell Adhesion Molecule-1 represented as VCAM-1; or a peptide having a QIDSPL sequence of VCAM-1.

5. The method according to any one of claims 1 to 4, wherein the contact step, the culture step, and the recovery step are repeated twice or more.

6. An instrument for separating megakaryocytes and platelets, the instrument comprising a substrate coated with a biocompatible polymer that adheres to megakaryocytes via at least one of a VLA-4 integrin or a VLA-5 integrin.

## INTERNATIONAL SEARCH REPORT

| International application No. |
|---|
| PCT/JP2018/010508 |

### A. CLASSIFICATION OF SUBJECT MATTER

Int.Cl. C07K1/14(2006.01)i, A61K35/19(2015.01)i, A61M1/02(2006.01)i, A61P7/00(2006.01)i, C12M1/00(2006.01)i, C07K14/78(2006.01)n, C12N15/09(2006.01)n

According to International Patent Classification (IPC) or to both national classification and IPC

### B. FIELDS SEARCHED

Minimum documentation searched (classification system followed by classification symbols)

Int.Cl. C07K1/14, A61K35/19, A61M1/02, A61P7/00, C12M1/00, C07K14/78, C12N15/09

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

JSTPlus/JMEDPlus/JST7580 (JDreamIII), CAplus/MEDLINE/EMBASE/BIOSIS (STN), WPIDS/WPIX (STN), AGRICOLA (STN), FSTA (STN), TOXCENTER (STN)

### C. DOCUMENTS CONSIDERED TO BE RELEVANT

| Category* | Citation of document, with indication, where appropriate, of the relevant passages | Relevant to claim No. |
|---|---|---|
| X | JP 2013-031428 A (THE UNIVERSITY OF TOKYO) 14 February 2013, claims, paragraphs [0037], [0051]–[0053] & JP 2017-99411 A | 1-6 |
| X | JP 2016-538859 A (PLATOD) 15 December 2016, claims, paragraph [0038] & US 2016/0272941 A1 & WO 2015/075030 A1: claims, page 9 & EP 3071327 A1 & AU 2014352015 A & CA 2930131 A & KR 10-2016-0086924 A & CN 105980057 A & IL 245714 D | 1-6 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30 May 2018 (30.05.2018) | 12 June 2018 (12.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**EP 3 597 660 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 09122747 A
- JP 2003093499 A
- WO 93024157 A
- JP 2000245833 A
- WO 2004110139 A
- EP 1014176 A2
- US 6992172 B
- WO 2004085473 A
- WO 2008103041 A
- JP 18010508 B
- JP 20180316 B
- JP 0002000635 A
- JP 1800558056 B